# EUROPÄISCHE PATENTSCHRIFT

(11) **EP 0 532 539 B1**
(45) Veröffentlichungstag und Bekanntmachung des Hinweises auf die Patenterteilung: **27.09.1995**
(21) Anmeldenummer: 91909843.4
(22) Anmeldetag: 21.05.1991
(51) Int. Cl.: C07C 303/32, C11D 1/28

(54) **HOCHKONZENTRIERTE PASTEN VON ALPHA-SULFOFETTSÄUREALKYLESTER-ALKALIMETALLSALZEN**
PROCESS FOR PRODUCING HIGHLY CONCENTRATED PASTES OF ALPHA-SULPHO FATTY ACID ALKYL ESTER ALKALI METAL SALTS
PATES TRES CONCENTREES DE SELS DE METAUX ALCALINS-ALKYLESTERS D'ACIDES GRAS ALPHA-SULFONES

(30) Priorität: 30.05.1990 DE 4017468
(43) Veröffentlichungstag der Anmeldung: 24.03.1993
(73) Patentinhaber: Henkel Kommanditgesellschaft auf Aktien, 40191 Düsseldorf (DE)
(72) Erfinder: COLIGNON, Dietmar, D-4006 Ekrath (DE); DORRA, Erich, D-4000 Düsseldorf 13 (DE); PANTHEL, Günter, D-5657 Haan (DE); SCHMIDT, Wolfgang, D-4019 Monheim (DE); WREDE, Norbert, D-4000 Düsseldorf 13 (DE)
(86) Internationale Anmeldenummer: EP9100939
(87) Internationale Veröffentlichungsnummer: WO9118870

(56) Entgegenhaltungen:
- EP-A- 0 182 118
- EP-A- 0 222 237

## Beschreibung

Die Erfindung betrifft ein Verfahren zur Herstellung frei beweglicher Pasten von α-Sulfofettsäurealkylester-Alkalimetallsalzen mit Waschaktivsubstanzgehalten von 60 bis 70 Gew.-%, bei dem man sauren α-Sulfofettsäurealkylester unter Einhaltung von definierten pH-Bedingungen mit wässrigen Alkalimetallhydroxidlösungen neutralisiert. Unter Waschaktivsubstanz (WAS) wird im Zusammenhang mit der Erfindung die Summe aus α-Sulfofettsäurealkylester-Alkalimetallsalz und dem stets als Nebenprodukt vorhandenen α-Sulfofettsäure-Dialkalimetallsalz im neutralisierten α-Sulfofettsäureester verstanden.

α-Sulfofettsäurealkylester-Alkalimetallsalze haben derzeit steigende Bedeutung als oberflächenaktive Substanzen für Wasch- und Reinigungsmittel aus nachwachsenden natürlichen Rohstoffen. Man erhält die α-Sulfofettsäurealkylester-Alkalimetallsalze nach bekannten Verfahren in Form von wässrigen Lösungen oder Pasten durch Neutralisation von α-Sulfofettsäurealkylestern, welch letztere durch Umsetzung von Fettsäureniedrigalkylestern mit gasförmigem SO₃ synthetisiert werden können. Als Basis für die Herstellung der α-Sulfofettsäurealkylester-Alkalimetallsalze dienen letzten Endes Fette und Öle natürlichen Ursprungs, aus denen die Fettsäureniedrigalkylester durch Fettspaltung und nachfolgende Veresterung der freien Fettsäuren mit niederen Alkanolen oder durch Umesterung der natürlichen Triglyceride mit niederen Alkanolen erhalten werden. Bei beiden Reaktionen ist Methanol als niederes Alkanol bevorzugt. Die Fettsäureniedrigalkylester stellen Gemische dar, in denen Fettsäurereste mit 6 bis 22 Kohlenstoffatomen vorkommen, wobei die Kettenlängenverteilung vom Ursprung der natürlichen Fette oder Öle abhängig ist. Diese Fettsäureestergemische werden häufig nicht als solche, sondern in Form bestimmter Fraktionen zur Synthese eingesetzt. Durch Sulfonierung der Fettsäureestergemische mit gasförmigem SO₃ erhält man saure α-Sulfofettsäurealkylester, die durch Neutralisation auf einen pH-Wert von 6 bis 8 in wässrige Pasten von α-Sulfofettsäurealkylester-Alkalimetallsalzen überführt werden. Die rohen α-Sulfofettsäurealkylester und gegebenenfalls auch ihre Alkalimetallsalze stellen mehr oder weniger gefärbte Produkte dar, die in der Regel vor und/oder nach der Neutralisation einer Behandlung mit üblichen Bleichmitteln wie Wasserstoffperoxid oder Alkalimetallhypochlorit unterworfen werden müssen.

Eine besondere Schwierigkeit bei der Herstellung und Handhabung von wässrigen α-sulfofettsäurealkylester-Alkalimetallsalzpasten ergibt sich aus ihrem Viskositätsverhalten in Abhängigkeit von der Feststoffkonzentration. Die nach den gängigen technischen Verfahren hergestellten α-Sulfofettsäurealkylester-Alkalimetallsalze, im Folgenden auch kurz als Estersulfonate bezeichnet, bilden in wässrigen Zusammensetzungen nur bei WAS-Gehalten bis zu etwa 40 Gew.-% und dann wieder ab WAS-Gehalten von etwa 55 Gew.-% Lösungen oder Suspensionen mit so geringer Viskosität, daß eine hinreichende Beweglichkeit gegeben ist, um einen störungsfreien Ablauf technischer Vorgänge zu gewährleisten. In dem dazwischen liegenden Konzentrationsbereich, also bei WAS-Gehalten von etwa 40 bis 55 Gew.-% zeigen die wässrigen Estersulfonat-Zusammensetzungen extrem hohe Viskositätswerte und stellen sich als mehr oder weniger feste Gele dar, die nicht gerührt oder umgepumpt werden können. Die Unter- und Obergrenzen der individuellen Viskositätsmaxima können im übrigen jeweils um ± 5 Gew.-%. Feststoffgehalt schwanken. Dieses besondere Konzentrations-/Viskosithätsverhalten bedingt, daß durch einfache Neutralisation der sauren α-Sulfofettsäurealkylester mit der berechneten Menge wässriger Alkalimetallhydroxidlösungen keine Estersulfonatpasten mit WAS-Gehalten über 35 bis 40 Gew.-% eingestellt werden können. Nach dem Überschreiten der Untergrenze des Viskositätsmaximums geht die Rührbarkeit und Vermischbarkeit des reagierenden Gemisches verloren. Die mangelnde Rühr- und Mischbarkeit verhindert einen hinreichenden schnellen Abtransport der Neutralisationswärme. Durch lokale Konzentrations- und Temperaturspitzen werden unerwünschte Nebenreaktionen ausgelöst, insbesondere die Spaltung der in den Estersulfonaten vorhandenen Esterbindungen, wodurch im Endprodukt unerwünscht hohe Konzentrationen an Alkalimetall-Disalzen der freien α-Sulfofettsäuren entstehen. Selbstverständlich ist auch die weitere Verarbeitung von Estersulfonatpasten, die durch den hohen Viskositätsanstieg immobilisiert sind, bis hin zur Undurchführbarkeit beeinträchtigt, allein schon durch die Tatsache, daß derartige wässrige Zusammmensetzungen nicht mehr gieß- und pumpfähig sind.

Das Entstehen von Disalzen der freien α-Sulfofettsäurealkylester ist aus mehreren Gründen unerwünscht. Die Disalze sind in Wasser nur im beschränkten Umfang löslich und zeigen darüber hinaus unzureichende oberflächenaktive Eigenschaften. Vor allem aber haben Disalze als Nebenprodukte in Estersulfonatpasten eine beträchtliche viskositätssteigernde Wirkung.

Es hat in der Vergangenheit nicht an Versuchen gefehlt, die durch das besondere Konzentrations-/Viskositätsverhalten der Estersulfonate und das unerwünschte Entstehen von α-Sulfofettsäure-Disalzen bedingten nachteiligen Erscheinugen wenigstens weitgehend ausschalten. So wurde vorgeschlagen, das Fließverhalten von wässrigen Estersulfonat-Zusammensetzungen durch den Zusatz von Fließhilfsmitteln zu verbessern. Nach der DE-OS 33 05 430 werden als Viskositätsregler aliphatische Alkohole mit 8 bis 40 Kohlenstoffatomen und 1 bis 6 Hydroxylgruppen, Alkylphenole und Anlagerungsprodukte von bis zu 20 Mol Ethylenoxid und/oder Propylenoxid an die genannten Alkohole und Alkylphenole eingesetzt.

Im Zusammenhang mit der unerwünschten Bildung von Disalzen bei der Aufarbeitung der saueren α-Sulfofettsäurealkylester beschreibt die DE-OS 31 23 681 ein Verfahren, bei dem die Neutralisation in zwei Stufen durchgeführt wird. In der ersten Neutralisationsstufe wird dort mit einer 15 bis 50 gew.-%igen Alkalimetallhydroxidlösung in Gegenwart eines Alkohols mit 1 bis 4 Kohlenstoffatomen, vorzugsweise Methanol, in einer Menge von 5 bis 20 Gew.-%, bezogen auf das Gewicht des sulfonierten Produktes, bis auf einen pH-Wert von 2,5 bis 4 neutralisiert, bevor in der zweiten Neutralisationsstufe mit Hilfe einer stärker verdünnten Alkalimetallhydroxidlösung ein End-pH-Wert von 6 bis 7 eingestellt wird. Mit Hilfe dieses Verfahrens soll es möglich sein, in den Estersulfonat-Zusammensetzungen den Disalzgehalt auf 5 Gew.-%, bezogen auf Waschaktivsubstanz, oder weniger zurückzudrängen. Ein gravierender Nachteil dieses Verfahrens liegt auf der Hand: Die auf diese Weise hergestellten Estersulfonatpasten enthalten beträchtliche Mengen Alkohol, die bei der Herstellung von Waschmittelgemischen durch Sprühtrocknung insofern stören, als sie das unerwünschte "Pluming" auslösen können. Zur Begrenzung des Alkoholgehaltes in den Endprodukten schlägt die DE-OS 33 34 517 vor, die gegebenenfalls durchgeführte Bleiche und die Neutralisation der rohen α-Sulfofettsäurealkylester in Gegenwart einer solchen Menge eines niederen Alkohols vorzunehmen, daß eine wässrige Aufschlämmung mit 30 bis 40 Gew.-% und - bezogen auf das Gewicht des α-Sulfofettsäureestersalzes - 5 bis 15 Gew.-% eines niederen Alkoholsulfates sowie 8 bis 40 Gew.-% des niederen Alkohols erhalten wird. Abschließend soll die wässrige Aufschlämmung derart eingeengt werden, daß sie 40 bis 65 Gew.-% α-Sulfofettsäureestersalz, 2 bis 10 Gew.-% niederes Alkoholsulfat und höchstens 2 Gew.-% niederen Alkohol enthält.

Nach der DE-OS 34 32 324 läßt sich der Disalzgehalt in α-Sulfofettsäurealkylester-Alkalimetallsalz-Pasten dadurch regeln und senken, daß man das rohe Sulfonierungsprodukt vor der Behandlung mit einem wässrigen Medium einer Umesterungsreaktion unterwirft, bei der - bezogen auf den zur α-Sulfonierung nicht verbrauchten SO₃-Anteil - wenigstens 0,5 Mol-Äquivalente Alkohol eingesetzt werden. Nach der DE-OS 35 38 910 können α-Sulfofettsäurealkylester-Salzpasten mit Feststoffgehalten oberhalb von 35 Gew.-% dadurch hergestellt werden, daß man die rohen Estersulfonate einer Umesterung im Sinne der DE-OS 34 32 324 unterwirft und dann bei der anschließenden Aufarbeitung durch Neutralisation mit oder ohne vorhergehender oder nachfolgender Bleiche in den wässrigen Pasten Feststoffgehalte von mehr als 35 Gew.-% einstellt.

Den Herstellungsverfahren für α-Sulfofettsäurealkylester-Alkalimetallsalzen, die bei der Aufarbeitung der rohen Sulfonierungsprodukte einen Zusatz von niederen Alkoholen vorsehen, haftet der Nachteil an, daß sie zu pastösen Zusammensetzungen führen, die mit den üblichen Bleichmitteln nicht mehr in hinreichendem Maß aufgehellt werden können.

Die vorliegende Erfindung geht auf die Aufgabenstellung zurück, ein Verfahren aufzufinden, das es gestattet, ohne Zusatz von Fremdstoffen wie längerkettigen aliphatischen Mono- und Polyalkoholen und deren Alkylenoxidanlagerungsprodukten, Alkylphenolen und deren Alkylenoxidanlagerungsprodukten oder kurzkettigen Alkoholen durch direkte Neutralisation der sauren α-Sulfofettsäurealkylester mit wässrigen Alkalimetallhydroxidlösungen zu fließ- und pumpfähigen Estersulfonatpasten mit WAS-Gehaltehalten von 60 bis 70 Gew.-% zu gelangen. Die Lehre der Erfindung geht von der überraschenden Erkenntnis aus, daß dieses Ziel erreicht werden kann, wenn man bei der Neutralisation das saure Sulfonierungsprodukt und die wässrige Alkalimetallhydroxidlösung in eine bereits vorliegende wässrige α-Sulfofettsäurealkylester-Alkalimetallsalzpaste einträgt und dabei gleichzeitig dafür Sorge trägt, daß der pH-Wert der wässrigen Phase sich nur in einem ganz bestimmten Bereich bewegt.

Gegenstand der Erfindung ist ein Verfahren zur Herstellung hochkonzentrierter Pasten von α-Sulfofettsäurealkylester-Alkalimetallsalzen mit guter Bleichbarkeit durch Umsetzung von Fettsäurealkylestern mit gasförmigem SO₃, anschließende Nachreaktion in flüssiger Phase und Neutralisation mit wässrigen Alkalihydroxidlösungen, bei dem man bei der Neutralisation das Sulfierprodukt und wässrige Alkalimetallhydroxid-Lösung unter Aufrechterhaltung eines pH-Wertes im Bereich von 2 bis 8 in eine anfänglich mindestens 55 Gew.-% α-Waschaktivsubstanz enthaltende wässrige Phase einleitet und Waschaktivsubstanzgehalte von 60 bis 70 Gew.-%, vorzugsweise 60 bis 65 Gew.-%, einstellt.

In einer speziellen Ausführungsform der Erfindung wird das Sulfierprodukt zusammen mit wässriger Alkalimetallhydroxidlösung in eine anfänglich 60 bis 70 Gew.-% Waschaktivsubstanz enthaltende wässrige Phase eingeleitet.

Zu Beginn der Neutralisation werden die Waschaktivsubstanz enthaltende Lösungen auf einen Wert im Bereich von 2 bis 8 eingestellt.

In einer bevorzugten Ausführungsform der Erfindung wird während des Neutralisationsvorgangs in der wässrigen Phase ein pH-Wert von 5 bis 8, vorzugsweise im Bereich von 5,5 bis 7,5 eingestellt und aufrechterhalten.

Der pH-Wert der wässrigen Phase wird vorzugsweise durch Variation der Volumenströme von α-Sulfofettsäurealkylester und wässriger Alkalimetallhydroxidlösung aufrechterhalten. Die Neutralisation der α-Sulfofettsäurealkylester wird zweckmäßigerweise bei Temperaturen unterhalb von 95 °C, vorzugsweise bei Temperaturen im Bereich von 60 bis 90 °C durchgeführt. Als Neutralisationsbase ist Natriumhydroxid bevorzugt.

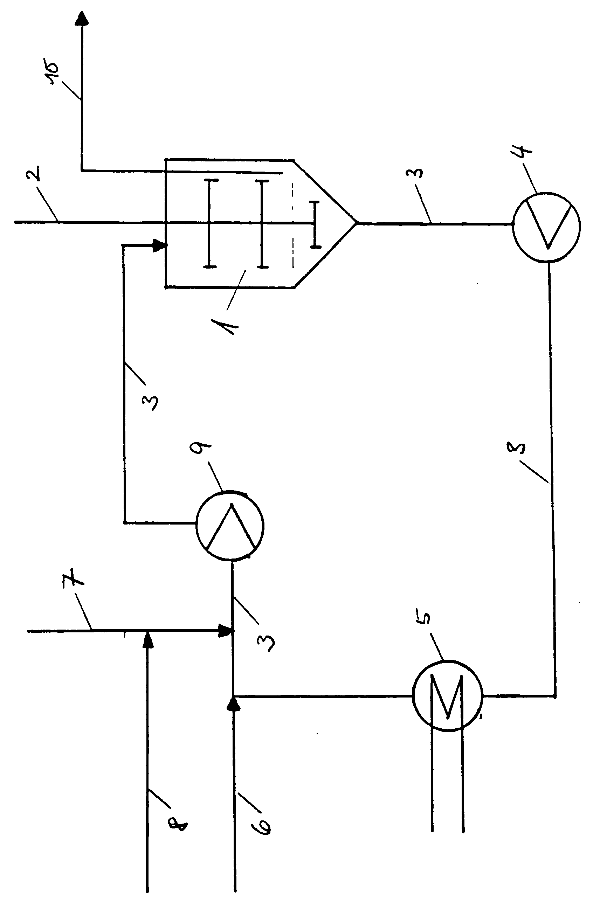

Die Neutralisation der sauren α-Sulfofettsäurealkylester wird zweckmäßigerweise in einer Neutralisationsschleife durchgeführt, deren schematischer Aufbau in der Fig. 1 dargestellt ist. Der überwiegende Teil der wässigen Phase befindet sich in dem Rührbehälter 1 und wird mit Hilfe des Rührers 2 ständig durchmischt. Über die Kreislaufleitung 3 wird daraus mittels der Umwälzpumpe 4 wässrige Phase laufend abgezogen und in dem für die Steuerung der Reaktionstemperatur vorgesehenen Kühler 5 im erforderlichen Ausmaß gekühlt. Der zu neutralisierende α-Sulfofettsäurealkylester wird über die Leitung 6 in den Strom der umgepumpten wässrigen Phase eingeleitet. Wässrige Alkalimetallhydroxidlösung einer Standard-Konzentration, beispielsweise 50 gew.-%ige Natriumhydroxidlösung, wird dem Kreislauf über die Leitung 7 zugeführt. Durch Zufuhr von Wasser über die Leitung 8 kann die Konzentration der Standard-Alkalimetallhydroxidlösung vor deren Einspeisung in den Produktkreislauf auf den akut geforderten Wert vermindert werden. Das Gemisch aus saurem α-Sulfofettsäurealkylester, Alkalimetallhydroxidlösung und im Kreislauf geführter wässriger Phase gelangt dann zur weiteren Homogenisierung in den Mischer 9 und wird von dort über den letzten Abschnitt der Kreislaufleitung 3 in den Rührbehälter 1 gefördert. Die bei der Neutralisation entstehende α-Sulfofettsäurealkylester-Alkalimetallsalzpaste kann über die Leitung 10 abgezogen werden. Eine Neutralisationsschleife des hier beschriebenen Typs kann ausschließlich aus üblichen Vorrichtungen, Armaturen und Leitungen aufgebaut werden. Die erforderliche Überwachung von pH und Reaktionstemperatur und die Regulierung der Produkt- und Kühlmittelströme kann nach bekannten Verfahren der Meß- und Regeltechnik für chemische Verfahren erfolgen.

Das erfindungsgemäße Verfahren kann in der beschriebenen Neutralisationsschleife durch geeignete Steuerung der maßgeblichen Parameter kontinuierlich durchgeführt werden. Bei der kontinuierlichen Ausgestaltung des Verfahrens wird über die Leitung 10 α-Sulfofettsäurealkylester-Natriumsalzpaste in dem Maß abgezogen wie über die Leitung 6 Sulfonierungsprodukt und die Leitung 7 Alkalimetallhydroxid zugeführt werden.

Die für die Durchführung des Verfahrens als wässrige Phase notwendigen mindestens 55 Gew.-% Waschaktivsubstanz enthaltenen Pasten können in der Neutralisationsschleife dadurch hergestellt werden, daß man in eine vorgegebene Menge Wasser unter Umpumpen der wässrigen Phase sauren α-Sulfofettsäurealkylester und die für dessen Neutralisation erforderliche Menge wässriger Alkalimetallhydroxidlösung unter Aufrechterhaltung eines pH-Wertes von 2 bis 8, vorzugsweise von 3 bis 5, und einer Temperatur unterhalb von 95 °C, vorzugsweise im Bereich von 60 bis 90 °C einleitet, bis in der wässrigen Phase der gewünschte Gehalt an Waschaktivsubstanz erreicht ist.

Unter Fettsäurealkylestern im Sinne der Erfindung werden Niedrigalkylester von gesättigten Fettsäuren verstanden, insbesondere Ester von Fettsäuren mit 10 bis 18 Kohlenstoffatomen und gesättigten aliphatischen Alkoholen mit 1 bis 4 Kohlenstoffatomen. Grundsätzlich kann man von einzelnen Fettsäurealkylestern ausgehen. In der Regel verwendet man als Ausgangsmaterial jedoch Estergemische, wie sie aus Fetten und Ölen natürlichen Ursprungs entweder durch Esterspaltung und nachfolgende Veresterung mit niederen Alkanolen oder durch Umesterung mit niederen Alkanolen nach bekannten Verfahren erhältlich sind, wobei die entsprechenden Fettsäuremetylestergemische wiederum bevorzugt sind. Soweit die auf diese Weise erhaltenen Fettsäureestergemische größere Anteile an Estern von Fettsäuren mit weniger als 10 Kohlenstoffatomen enthalten, werden diese "Vorlauffettsäureester" in der Regel durch Destillation abgetrennt. Die Fettsäureester dürfen außer der CH₂-Gruppe in α-Stellung zur Estergruppierung keine sulfatierbaren oder sulfonierbaren Gruppen enthalten. Aus diesem Grund kommen Hydroxyfettsäureester oder Hydroxyfettsäureester enthaltende Gemische nicht als Ausgangsmaterial in Betracht. Fettsäureestergemische, die nicht zu vernachlässigende Mengen an Estern ungesättigter Fettsäuren enthalten, insbesondere solche Ester, die eine Jodzahl über 5 aufweisen, sind erst nach einer Absättigung der Doppelbindungen im Zuge einer hydrierenden Härtung nach bekannten Verfahren als Ausgangsmaterial geeignet. Bei der hydrierenden Härtung werden die Jodzahlwerte der Estergemische vorzugsweise auf Werte von 0,2 und kleiner vermindert.

Die Sulfonierung der Fettsäureester erfolgt mit gasförmigem SO₃ als Sulfonierungsreagenz bei Temperaturen von 30 bis 80 °C. Dabei wird das SO₃ mit Luft oder Stickstoff verdünnt, vorzugsweise in Form eines Gasgemisches mit 1 bis 10 Vol.-% SO₃ mit den Fettsäureestern in Berührung gebracht. Die Menge des SO₃ wird so bemessen, daß das Sulfonierungsmittel in einem molaren Überschuß von mindestens 10 % vorliegt. Vorzugsweise liegt das Molverhältnis von Fettsäureester : SO₃ im Bereich von 1 : 1,2 bis 1 : 1,8 liegt. Diese Umsetzung kann in üblichen, für die Sulfonierung von organischen Verbindungen wie Fettalkoholen, Alkylbenzolen oder Olefinen geeigneten Reaktoren, insbesondere in Fallfilmreaktoren oder mehrstufigen Rührkesselkaskaden durchgeführt werden.

Das aus dem Sulfierreaktor kommende rohe Sulfonierungsprodukt weist noch nicht den gewünschten Sulfonierungsgrad auf. Aus diesem Grund wird das rohe Reaktionsprodukt unmittelbar nach der Sulfonierung einer geeigneten Vorrichtung zugeführt, in der es unter mechanischer Bewegung über einen Zeitraum von 20 bis 40 Minuten, vorzugsweise 25 bis 35 Minuten, einer temperaturkontrollierten Nachreaktion überlassen wird, bis der gewünschte Sulfiergrad erreicht ist. Die für diesen Reaktionsschritt notwendige Vorrichtung kann aus einem üblichen Reaktor mit Heiz- und Kühlkreislauf, einer üblichen temperierbaren Rohrschlange oder einer üblichen Rührkesselkaskade bestehen. Die Nachreaktion wird bei Temperaturen von 60 bis 100 °C durchgeführt. Die mechanische Bewegung des sulfonierten Produktes während der Nachreaktion kann durch Rühren, durch Zufuhr des Produktes unter Druck, durch den Einbau von Umlenkschikanen in die Vorrichtung oder, bei Verwendung einer Rohrschlange, durch Ausbildung einer turbulenten Strömung bewirkt werden. Die Nachreaktion des sulfonierten Produktes kann durch geeignete Wahl der genannten Parameter, insbesondere der Reaktionszeit, so gesteuert werden, daß ein Sulfiergrad von mindestens 90 %, vorzugsweise 94 bis 98 % erreicht wird.

Im Anschluß an die Nachreaktion wird das gealterte Sulfonierungsprodukt der Neutralisation gemäß der Erfindung zugeführt.

Die bei der Neutralisation erhaltenen Estersulfonatpasten stellen mehr oder weniger stark gefärbte Substanzen dar, die vor ihrer weiteren Verarbeitung gebleicht werden müssen. Die Bleichung erfolgt in an sich bekannter Weise unter Verwendung üblicher Bleichmittel wie wässrigen Lösungen von Natriumhypochlorit oder, vorzugsweise, wässrigen Lösungen von Wasserstoffperoxid. Die Bleichung mit Wasserstoffperoxid erfolgt bei pH-Werten im sauren Bereich. Vorzugsweise wird die Wasserstoffperoxidbleichung bei pH-Werten oberhalb von 5 durchgeführt.

### Beispiel

Als Ausgangsmaterial wurde ein technischer Palmitin-/Stearinsäuremethylester (in Gew.-% nach der Kettenlänge im Fettsäurerest: 0,2 C₁₂; 1,2 C₁₄; 61,4 C₁₆; 0,9 C₁₇; 35,9 C₁₈; 0,4 C₂₀; mittleres Molgewicht 281,5; Säurezahl 1,1; Jodzahl 0,1; Verseifungszahl 202,1) verwendet. Der Fettsäuremethylester wurde kontinuierlich in einem üblichen Fallfilmreaktor bei 80 °C mit einem SO₃-Luftgemisch (5 Vol.-% SO₃) im Molverhältnis 1 : 1,25 sulfoniert. Das resultierende Reaktionsgemisch wurde in einer aus vier Rührkesseln bestehenden Verweilzeitkaskade mit einer Verweilzeit von 25 Minuten einer Nachreaktion unterworfen. Danach betrug die Säurezahl des Sulfonierungsproduktes 198. Der Sulfonierungsgrad lag bei 96 %.

In eine Neutralisationsschleife des beschriebenen Typs wurden 1448 kg einer Estersulfonatpaste mit einem WAS-Gehalt von 62,5 Gew.-% (52,0 Gew.-% α-Sulfofettsäuremethylester-Natriumsalz und 10,5 Gew.-% α-Sulfofettsäuredinatriumsalz; pH 7,5) eingebracht und anschließend als wässrige Phase umgepumpt. In die im Kreislauf geführte wässrige Phase wurde das vorstehend beschriebene gealterte Sulfonierungsprodukt und 25 gew.-%ige wässrige Natriumhydroxidlösung mit einer solchen Geschwindigkeit eingespeist, daß in der wässrigen Phase ein pH-Wert von 7,5 aufrecht erhalten blieb. Dabei wurden auf 1 kg Sulfonierungsprodukt 0,53 kg 25 gew.-%ige Natriumhydroxidlösung dem Neutralisationskreislauf zugeführt. Nachdem sich die wässrige Phase durch die freiwerdende Neutralisationswärme auf etwa 75 °C erwärmt hatte, wurde die Reaktionstemperatur mit Hilfe des Kühlers auf diesem Wert gehalten. Von diesem Zeitpunkt an wurde neutralisiertes Produkt in da Maß aus dem Rührbehälter abgezogen, wie Sulfonierungsprodukt und Natriumhydroxidlösung dem Neutralisationskreislauf zugeführt wurden. Der Neutralisationskreislauf wurde auf diese Weise über mehrere Tage im stationären Zustand betrieben. Die wässrige Phase konnte während der ganzen Zeit ohne Schwierigkeiten gerührt und umgepumpt werden.

Die laufend abgezogene α-Sulfofettsäuremethylester-Natriumsalzpaste hatte einen WAS-Gehalt von 62,5 ± 2,5 Gew.-%. Die Zusammensetzung der Waschaktivsubstanz stimmte innerhalb eines gewissen Schwankungsbereichs mit derjenigen der anfangs als wässrige Phase eingesetzten Paste überein.

Die ursprünglich als wässrige Phase eingesetzte α-Sulfofettsäuremethylester-Natriumsalzpaste war auf folgendem Weg hergestellt worden:
In die Neutralisationsschleife des beschriebenen Typs wurden 467 kg Wasser eingebracht und umgepumpt. In den Kreislauf der wässrigen Phase wurden 948 kg des vorstehend beschriebenen gealterten Sulfonierungsproduktes und 250 kg 50 gew.-%igen Natriumhydroxidlösung zunächst mit einer solchen Geschwindigkeit eingespeist, daß in der wässrigen Phase ein pH-Wert von 4,5 bis 5,5 aufrechterhalten wurde. Sobald der WAS-Gehalt der wässrigen Phase 55 Gew.-% erreicht hatte, wurden die Zuflußgeschwindigkeiten des Sulfonierungsproduktes und der Natriumhydroxidlösung so einreguliert, daß der pH-Wert in der wässrigen Phase bei 6 lag. Nachdem das gesamte saure Sulfonierungsprodukt dem Neutralisationskreislauf zugeführt war, wurde der pH-Wert der wässrigen Phase durch Zugabe der restlichen Menge Natriumhydroxidlösung auf 7,5 angehoben. Während der gesamten Neutralisation wurde die Reaktionstemperatur auf 70 bis 85 °C gehalten.

## Patentansprüche

1. Verfahren zur Herstellung hochkonzentrierter Pasten von α-Sulfofettsäurealkylester-Alkalimetallsalzen mit guter Bleichbarkeit durch Umsetzung von Fettsäurealkylestern mit gasförmigen SO₃, anschließende Nachreaktion in flüssiger Phase und Neutralisation mit wässrigen Alkalihydroxidlösungen, dadurch gekennzeichnet, daß man bei der Neutralisation das Sulfierprodukt und wässrige Alkalimetallhydroxidlösung unter Aufrechterhaltung eines pH-Wertes im Bereich von 2 bis 8 in eine anfänglich mindestens 55 Gew.-% Waschaktivsubstanz enthaltende wässrige Phase einleitet und Waschaktivsubstanzgehalte von 60 bis 70 Gew.-%, vorzugsweise 60 bis 65 Gew.-%, einstellt.

2. Verfahren nach Anspruch 1, dadurch gekennzeichnet, daß man das Sulfierprodukt zusammen mit wässriger Alkalimetallhydroxidlösung in eine anfänglich 60 bis 70 Gew.-% Waschaktivsubstanz enthaltende wässrige Phase einleitet.

3. Verfahren nach mindestens einem der Ansprüche 1 und 2, dadurch gekennzeichnet, daß man in der wässrigen Phase einen pH-Wert von 5 bis 8, vorzugsweise einen pH-Wert im Bereich von 5,5 bis 7,5, aufrecht erhält.

4. Verfahren nach mindestens einem der Ansprüche 1 bis 3, dadurch gekennzeichnet, daß man den pH-Wert der wässrigen Phase durch Variation der Volumenströme von α-Sulfofettsäurealkylester und wässriger Alkalimetallhydroxidlösung aufrecht erhält.

5. Verfahren nach mindestens einem der Ansprüche 1 bis 4, dadurch gekennzeichnet, daß man die Neutralisation bei Temperaturen unterhalb von 95 °C, vorzugsweise bei Temperaturen im Bereich von 60 bis 90 °C, durchführt.

6. Verfahren nach mindestens einem der Ansprüche 1 bis 5, dadurch gekennzeichnet, daß man als Ausgangsmaterial Fettsäuremethylester einsetzt.

7. Verfahren nach mindestens einem der Ansprüche 1 bis 6, dadurch gekennzeichnet, daß man als Ausgangsmaterial durch Umesterung von natürlichen Fetten und/oder Ölen mit Methanol erhältliche Fettsäuremethylestergemische einsetzt.

8. Verfahren nach mindestens einem der Ansprüche 1 bis 7, dadurch gekennzeichnet, daß man die Fettsäurealkylester in einem üblichen Sulfierreaktor mit einem mindestens 10 %-igen molaren Überschuß an SO₃ zur Umsetzung bringt.

9. Verfahren nach mindestens einem der Ansprüche 1 bis 8, dadurch gekennzeichnet, daß man als Sulfierreagenz ein SO₃/Luft- oder SO₃/Stickstoffgemisch mit 1 bis 10 Gew.-% SO₃ einsetzt.

10. Verfahren nach mindestens einem der Ansprüche 1 bis 9, dadurch gekennzeichnet, daß man die Umsetzung mit SO₃ in einem Molverhältnis Fettsäureester : SO₃ im Bereich von 1 : 1,2 bis 1 : 1,8 durchführt.

11. Verfahren nach mindestens einem der Ansprüche 1 bis 10, dadurch gekennzeichnet, daß man die Umsetzung mit SO₃ in einem Fallfilmreaktor oder in einer mehrstufigen Sulfierkaskade durchführt.

12. Verfahren nach mindestens einem der Ansprüche 1 bis 11, dadurch gekennzeichnet, daß man das aus dem Sulfierreaktor kommende rohe Sulfierprodukt in einer geeigneten Vorrichtung unter mechanischer Bewegung einer temperaturkontrollierten Nachreaktion bis zum Erreichen des gewünschten Sulfiergrades überläßt.

13. Verfahren nach mindestens einem der Ansprüche 1 bis 12, dadurch gekennzeichnet, daß man die Nachreaktion in einem Reaktor mit Heiz- und Kühlkreislauf, einer temperierbaren Rohrschlange oder einer Rührkesselkaskade durchführt.

14. Verfahren nach mindestens einem der Ansprüche 1 bis 9, dadurch gekennzeichnet, daß man die Nachreaktion bei 60 bis 100 °C durchführt.

15. Verfahren nach mindestens einem der Ansprüche 1 bis 10, dadurch gekennzeichnet, daß man bei der Nachreaktion die mechanische Bewegung des Sulfierproduktes durch Rühren, Zufuhr des Produktes unter Druck, Einbau von Umlenkschikanen in die Vorrichtung oder Ausbildung einer turbulenten Strömung in der Rohrschlange bewirkt.

16. Verfahren nach mindestens einem der Ansprüche 1 bis 11, dadurch gekennzeichnet, daß man das Sulfierprodukt so lange nachreagieren läßt, bis der Sulfiergrad mindestens 90 %, vorzugsweise 94 bis 98 % beträgt.

17. Verfahren nach mindestens einem der Ansprüche 1 bis 16, dadurch gekennzeichnet, daß man die erhaltenen α-Sulfofettsäurealkylester-Alkalimetallsalz-Pasten durch Bleichen weiterbehandelt.

18. Verfahren nach mindestens einem der Ansprüche 1 bis 17, dadurch gekennzeichnet, daß man die Pasten einer Bleiche unter Verwendung wässriger Lösungen von Wasserstoffperoxid unterwirft.

19. Verfahren nach mindestens einem der Ansprüche 1 bis 18, dadurch gekennzeichnet, daß man die Bleichung bei einem pH-Wert oberhalb von 5,0 durchführt.

## Claims

1. A process for the production of highly concentrated, readily bleachable pastes of α-sulfofatty acid alkyl ester alkali metal salts by reaction of fatty acid alkyl esters with gaseous SO₃, subsequent after-reaction in liquid phase and neutralization with aqueous alkali hydroxide solutions, characterized in that the sulfonation product and aqueous alkali metal hydroxide solution are introduced during neutralization into an aqueous phase initially containing at least 55% by weight α-washing-active substance at a pH value in the range from 2 to 8 and washing-active substance contents of 60 to 70% by weight and preferably 60 to 65% by weight are established.

2. A process as claimed in claim 1, characterized in that the sulfonation product and the aqueous alkali metal hydroxide solution are introduced into an aqueous paste initially containing 60 to 70% by weight washing-active substance.

3. A process as claimed in at least on of claims 1 and 2, characterized in that a pH value in the range from 5 to 8 and preferably in the range from 5.5 to 7.5 is maintained in the aqueous phase.

4. A process as claimed in at least one of claims 1 to 3, characterized in that the pH value of the aqueous phase is maintained by variation of the feed rates of α-sulfofatty acid alkyl ester and aqueous alkali metal hydroxide solution.

5. A process as claimed in at least one of claims 1 to 4, characterized in that the neutralization is carried out at temperatures below 95°C and preferably at temperatures in the range from 60 to 90°C.

6. A process as claimed in at least one of claims 1 to 5, characterized in that fatty acid methyl ester is used as the starting material.

7. A process as claimed in at least one of claims 1 to 6, characterized in that fatty acid methyl ester mixtures obtainable by transesterification of natural fats and/or oils with methanol are used as the starting material.

8. A process as claimed in at least one of claims 1 to 7, characterized in that the fatty acid alkyl esters are reacted with an at least 10% molar excess of SO₃ in a standard sulfonation reactor.

9. A process as claimed in at least one of claims 1 to 8, characterized in that an SO₃/air mixture or an SO₃/nitrogen mixture containing 1 to 10% by weight SO₃ is used as the sulfonation reagent.

10. A process as claimed in at least one of claims 1 to 9, characterized in that the reaction with SO₃ is carried out in a molar ratio of fatty acid ester to SO₃ of 1 : 1.2 to 1 : 1.8.

11. A process as claimed in at least one of claims 1 to 10, characterized in that the reaction with SO₃ is carried out in a falling film reactor or in a multiple-stage sulfonation cascade.

12. A process as claimed in at least one of claims 1 to 11, characterized in that the crude sulfonation product coming from the sulfonation reactor is subjected to a temperature-controlled after-reaction in a suitable apparatus, in which it is mechanically agitated, until the desired degree of sulfonation is reached.

13. A process as claimed in at least one of claims 1 to 12, characterized in that the after-reaction is carried out in a reactor comprising a heating and cooling circuit, a temperature-controlled pipe coil or a cascade of stirred tanks.

14. A process as claimed in at least one of claims 1 to 9, characterized in that the after-reaction is carried out at 60 to 100°C.

15. A process as claimed in at least one of claims 1 to 10, characterized in that the sulfonation product is mechanically agitated during the after-reaction by stirring, by introduction of the product under pressure, by installation of the chicane-like baffles in the apparatus or by generation of a turbulent flow in the pipe coil.

16. A process as claimed in at least one of claims 1 to 11, characterized in that the sulfonation product is left to after-react until the degree of sulfonation is at least 90% and preferably 94 to 98%.

17. A process as claimed in at least one of claims 1 to 16, characterized in that the α-sulfofatty acid alkyl ester alkali metal salt pastes obtained are further treated by bleaching.

18. A process as claimed in at least one of claims 1 to 17, characterized in that the pastes are bleached using aqueous solutions of hydrogen peroxide.

19. A process as claimed in at least one of claims 1 to 18, characterized in that bleaching is carried out at a pH above 5.0.

## Revendications

1. Procédé de fabrication de pâtes très concentrées de sels de métaux alcalins d'alkylesters d'acides gras α-sulfoniques présentant une bonne "blanchissabilité" par mise en réaction d'alkylesters d'acides gras avec du SO₃ gazeux, réaction postérieure en phase liquide et neutralisation par des solutions aqueuses d'hydroxydes de métaux alcalins, caractérisé en ce que l'on introduit, lors de la neutralisation, le produit de sulfuration et la solution aqueuse d'hydroxyde de métal alcalin, en respectant un pH compris dans l'intervalle de 2 à 8, dans une phase aqueuse contenant initialement au moins 55 % en poids de substance active-détergente, et en ajustant une concentration de substance active-détergente de 60 à 70 % en poids, de préférence de 60 à 65 % en poids.

2. Procédé selon la revendication 1, caractérisé en ce que l'on introduit le produit de sulfuration conjointement avec la solution aqueuse d'hydroxyde de métal alcalin, dans une phase aqueuse renfermant initialement 60 à 70 % en poids de substance active-détergente.

3. Procédé selon une des revendications 1 et 2, caractérisé en ce que l'on maintient dans la phase aqueuse un pH dans l'intervalle de 5 à 8, de préférence de 5,5 à 7,5.

4. Procédé selon au moins une des revendications 1 à 3, caractérisé en ce que l'on maintient le pH de la phase aqueuse en faisant varier le débit de l'alkylester d'acide gras α-sulfonique et de la solution aqueuse d'hydroxyde de métal alcalin.

5. Procédé selon au moins une des revendications 1 à 4, caractérisé en ce que l'on procède à la neutralisation à des températures inférieures à 95 °C, de préférence comprises dans l'intervalle de 60 à 90 °C.

6. Procédé selon au moins une des revendications 1 à 5, caractérisé en ce que l'on utilise comme matériel de départ, de l'ester méthylique d'acide gras.

7. Procédé selon au moins une des revendications 1 à 6, caractérisé en ce que l'on utilise comme matériel de départ, des mélanges d'esters méthyliques d'acides gras obtenables par transestérification de graisses et/ou d'huiles naturelles avec du méthanol.

8. Procédé selon au moins une des revendications 1 à 7, caractérisé en ce que l'on fait réagir les alkylesters d'acides gras dans un réacteur de sulfuration usuel avec un excès molaire de SO₃ d'au moins 10 %.

9. Procédé selon au moins une des revendications 1 à 8, caractérisé en que l'on utilise comme réactif de sulfuration, un mélange SO₃/air ou SO₃/azote contenant 1 à 10 % en poids de SO₃.

10. Procédé selon au moins une des revendications 1 à 9, caractérisé en que l'on opère la réaction avec le SO₃ avec un rapport molaire entre l'ester d'acide gras et le SO₃ situé dans l'intervalle de 1:1,2 à 1:1,8.

11. Procédé selon au moins une des revendications 1 à 10, caractérisé en que l'on opère la réaction avec le SO₃ dans un réacteur à ruissellement ou dans une cascade de sulfuration à plusieurs étages.

12. Procédé selon au moins une des revendications 1 à 11, caractérisé en que l'on soumet le produit de sulfuration brut provenant du réacteur de sulfuration à une réaction postérieure thermostatisée, dans un dispositif approprié, sous agitation mécanique, jusqu'à obtention du degré de sulfuration souhaité.

13. Procédé selon au moins une des revendications 1 à 12, caractérisé en que l'on opère la réaction postérieure dans un réacteur comportant un circuit de chauffage et de refroidissement, un serpentin thermostatisable ou une cascade de bacs à agitation.

14. Procédé selon au moins une des revendications 1 à 9, caractérisé en que l'on opère la réaction postérieure à une température comprise entre 60 et 100 °C.

15. Procédé selon au moins une des revendications 1 à 10, caractérisé en que l'on produit le mouvement mécanique du produit sulfoné au cours de la réaction postérieure, par agitation, amenée du produit sous pression, aménagement de chicanes dans le dispositif ou création d'un courant turbulent dans le serpentin.

16. Procédé selon au moins une des revendications 1 à 11, caractérisé en que l'on laisse le produit de sulfuration continuer & réagir jusqu'à ce que le degré de sulfuration atteigne au moins 90 %, de préférence 94 à 98 %.

17. Procédé selon au moins une des revendications 1 à 16, caractérisé en que l'on soumet ultérieurement à un blanchiment les pâtes de sels de métauxalcalins d'alkylesters d'acides gras α-sulfoniques obtenues.

18. Procédé selon au moins une des revendications 1 à 17, caractérisé en que l'on soumet les pâtes à un blanchiment en utilisant des solutions aqueuses d'eau oxygénée.

19. Procédé selon au moins une des revendications 1 à 18, caractérisé en que l'on opère le blanchiment à un pH supérieur à 5,0.
